# EUROPEAN PATENT SPECIFICATION

(11) **EP 4 268 853 B1**
(45) Date of publication and mention of the grant of the patent: **29.04.2026**
(21) Application number: 21914318.7
(22) Date of filing: 28.12.2021
(51) Int. Cl.: A61L 31/06, A61L 31/02, A61L 31/12, A61L 31/14

(54) **ORTHOPAEDIC INTERNAL FIXATION IMPLANTED MEDICAL DEVICE**
IMPLANTIERTE MEDIZINISCHE VORRICHTUNG ZUR ORTHOPÄDISCHEN INTERNEN FIXIERUNG
DISPOSITIF MÉDICAL IMPLANTABLE POUR FIXATION INTERNE ORTHOPÉDIQUE

(30) Priority: 28.12.2020 CN 202011606661
(43) Date of publication of application: 01.11.2023
(73) Proprietor: Biotyx Medical (Shenzhen) Co., Ltd., Shenzhen, Guangdong 518000 (CN)
(72) Inventor: ZHANG, Deyuan, Shenzhen, Guangdong 518000 (CN); QI, Haiping, Shenzhen, Guangdong 518000 (CN)
(74) Representative: Prinz & Partner mbB
(86) International application number: PCT/CN2021/141841
(87) International publication number: WO 2022/143582

(56) References cited:
- EP-A1- 3 157 590
- EP-A1- 3 378 504
- AU-A1- 2006 352 228
- CN-A- 102 014 798
- CN-A- 106 474 545
- CN-A- 106 693 043
- CN-A- 109 152 865
- TW-B- I 626 957
- US-A1- 2018 326 127
- NURIZZATI MOHD DAUD ET AL: "Degradation and in vitro cell-material interaction studies on hydroxyapatite-coated biodegradable porous iron for hard tissue scaffolds", JOURNAL OF ORTHOPAEDIC TRANSLATION, vol. 2, no. 4, 1 October 2014 (2014-10-01), pages 177 - 184, XP055594891, ISSN: 2214-031X, DOI: 10.1016/j.jot.2014.07.001

## Description

### Technical Field

The present invention relates to the technical field of medical devices, and in particular to an orthopedic internal fixation implant medical device.

### Background Art

Traditional orthopedic internal fixation devices are generally made of permanent metals such as stainless steel, titanium-based alloy, and cobalt-based alloy. These materials have excellent mechanical properties and biocompatibility, but they may have problems such as erosion, allergy, and osteoporosis due to the stress shielding effect after long-term retention in the human body. This requires a second surgery to remove the fracture after the patient has healed, which greatly increases the patient's pain and economic burden. Therefore, absorbable orthopedic fixtures made of degradable biomedical materials have been extensively studied for clinical use in recent years. The most attractive advantages of absorbable orthopedic internal fixation materials over permanent metal internal fixtures are that they do not require second surgical removal, greatly reducing the patient's pain.

Currently, absorbable orthopedic fixation materials mainly include absorbable polymers, magnesium, and alloys thereof.

Magnesium alloy orthopedic internal fixation materials, with good biocompatibility, can be degraded in vivo to avoid the pain of second surgical removal. Moreover, magnesium ions released by the materials can also promote the proliferation and differentiation of bone cells and promote bone growth and healing. The elastic modulus of magnesium alloy is close to that of human bone, which can effectively reduce the stress shielding effect. Meanwhile, the mechanical properties, such as tensile strength, of magnesium alloy are much higher than that of degradable polymer materials in clinical application, which can better meet clinical needs. However, the mechanical properties of magnesium-based alloy still do not reach the level of permanent metal implant materials, and the range of clinical application is limited. Magnesium-based alloy is still difficult to be used in the load-bearing parts, and can only be used in the non-load-bearing and less active positions. Secondly, magnesium-based alloy, with a faster degradation rate, will lose effective supporting and fixing function prematurely in response to implanting into the medical device. During the degradation, it will increase the local pH of the implantation site and generate excessive hydrogen bubbles, which are not conducive to the healing of the bone injury site.

US 2018/326127 A1 discloses an iron-based alloy absorbable and implantable medical device comprising an iron-based alloy substrate and a degradable polymer applied on said substrate, wherein the mass ratio of the alloy to the polymer is between 1:4 and 4:1. The weight-average molecular weight of the degradable polymer is in the range of 150 to 3,000 kDa and the degradable polymer may be a degradable polyester, or a mixture of the degradable polyester and at least one of a degradable polyanhydride, degradable polyamino acid and degradable polyphosphate ester, or a copolymer of at least one monomer forming the degradable polyester and at least one monomer forming the degradable polyanhydride, the degradable polyamino acid or the degradable polyphosphate ester.

EP 3 378 504 A1 shows an absorbable iron-based alloy implanted medical device comprising an iron-based alloy substrate, an alkaline protector disposed on the substrate, and a degradable polymer disposed on the surface of the substrate and/or the alkaline protector.

Mohd Daud et al. ("Degradation and in vitro cell-material interaction studies on hydroxyapatite-coated biodegradable porous iron for hard tissue scaffolds is a scientific paper discussing the effect of hydroxyapatite and hydroxyapatite/polycaprolactone coatings on porous iron scaffolds", Journal of Orthopaedic Translation (2014), 2, 177-184, doi: 10.1016/j .jot.2014.07.001) discusses the effect of hydroxyapatite and hydroxyapatite/polycaprolactone coatings on porous iron scaffolds.

Absorbable polymers, such as polylactic acid and polycaprolactone, have good biocompatibility, and a lot of clinical data have been accumulated. Polylactic acid with high molecular weight is melted and processed into shape, so as to make an orthopedic internal fixation implanted medical device with certain mechanical strength. Compared with the traditional permanent metal materials, the disadvantages of absorbable polymers are as follows: (1) Due to poor osteoconductivity and slow rate of repair of bone defects, it is difficult to achieve complete bone repair for larger bone defects; (2) Due to poor mechanical properties and insufficient mechanical strength, they are generally not applicable to the bearing parts (such as limbs), and only applicable to the fixation of cancellous bone, joint bone or less active bone in various non-bearing parts; (3) Due to fast early degradation rate, they cannot meet the mechanical property requirements before new bone tissue grows out; (4) The degradation of polylactic acid will generate an acidic environment, which will easily lead to serious inflammatory reactions at the implantation site. These drawbacks have greatly limited the application of absorbable polymer-based internal fixation implant medical devices. There is still room for improvement in the local slightly acidic environment generated by polylactic acid and in the osteoinductive capacity.

### Summary of the Invention

The scope of the invention is defined by the claims. Any references in the description to methods of treatment refer to the products of the present invention for use in a method for treatment.

An object of the present invention is to provide an orthopedic internal fixation implant medical device with better mechanical properties, better control of local pH values, reduction of local inflammatory reactions, controllable degradation rate, and induction of bone healing.

The object of the invention is solved by an orthopedic fixation implanted medical device according to claim 1.

According to a first aspect of the present invention, there is provided an orthopedic internal fixation implant medical device including an iron matrix and a filling material, the filling material including polylactic acid and an alkaline substance, where the polylactic acid has a weight-average molecular weight of M_{w} kDa, the alkaline substance includes a metal element, a mass ratio of the metal element in the alkaline substance to the polylactic acid is p, and the p and the M_{w} satisfy a formula of 2 M_{w}^-0.8 ≤ p ≤ 30 M_{w}^-0.5. The alkaline substance is a combination of hydroxyapatite and at least one of magnesium, magnesium alloy, zinc, zinc alloy, magnesium oxide, magnesium hydroxide, zinc oxide, zinc hydroxide, magnesium carbonate, zinc carbonate, magnesium phosphate, zinc phosphate, sodium carbonate, sodium bicarbonate, calcium oxide, calcium hydroxide, calcium carbonate, and calcium phosphate. That is, the alkaline substance includes hydroxyapatite, and also includes at least one of magnesium, magnesium alloy, zinc, zinc alloy, magnesium oxide, magnesium hydroxide, zinc oxide, zinc hydroxide, magnesium carbonate, zinc carbonate, magnesium phosphate, zinc phosphate, sodium carbonate, sodium bicarbonate, calcium oxide, calcium hydroxide, calcium carbonate, and calcium phosphate.

The iron matrix in the above orthopedic internal fixation implant medical device can provide sufficient mechanical support, which solves the problem of insufficient mechanical properties of polylactic acid orthopedic internal fixation medical devices and magnesium alloy orthopedic internal fixation medical devices. The filling material includes polylactic acid and an alkaline substance, where the alkaline substance can neutralize the acidity of the degradation product of the polylactic acid in the early stage, and a mass ratio of the metal element in the alkaline substance to the polylactic acid is set as p, the polylactic acid has a molecular weight of Mw kDa, and the p and the Mw satisfy a formula of 2 M_{w}^-0.8 ≤ p ≤ 30 M_{w}^-0.5. In response to satisfying the formula, it can not only keep the local pH stable and make the pH neutral to reduce the inflammatory reactions, which is beneficial to bone repair, but also slow down the early degradation rate of the iron matrix to maintain good mechanical properties during bone healing.

In an embodiment, the polylactic acid has a weight-average molecular weight of 5 kDa to 1000 kDa.

In an embodiment, the alkaline substance is one or more of a powder, granule, block or rod.

In an embodiment, a mass of the hydroxyapatite is 1% to 10% of that of the orthopedic internal fixation medical device.

In an embodiment, the polylactic acid is poly-dl-lactic acid or poly-L-lactic acid.

In an embodiment, the iron matrix is a hollow structure, and the filling material is filled inside the iron matrix; or the iron matrix is a mesh skeleton structure, and the filling material is filled in meshes of the mesh skeleton structure; or the iron matrix is a hollow space skeleton structure, and the filling material is filled inside the hollow space of the iron matrix; or a groove or hole is disposed on a surface of the iron matrix, and the filling material is filled in the groove or hole of the iron matrix; or the filling material is coated on the surface of the iron matrix.

In an embodiment, the orthopedic internal fixation implant medical device is a bone nail, bone plate, bone rod, or bone mesh.

In an embodiment, the iron matrix is pure iron, low alloy steel, or iron-based alloy with a carbon content of not more than 2.5 wt.%.

### Brief Description of the Drawings

Various other advantages and benefits will become apparent to the ordinarily skilled in the art upon reading the following detailed description of the preferred implementations. Accompanying drawings are only for the purposes of illustrating the preferred implementations and are not to be construed as limiting the present invention. And throughout the accompanying drawings, the same components are represented by the same reference numerals. In which:
Fig. 1 is a diagram of a cross-section of an orthopedic internal fixation implant medical device provided in Embodiment 1.
Fig. 2 is a diagram of a cross-section of an orthopedic internal fixation implant medical device provided in Embodiment 3.
Fig. 3 is a diagram of a cross-section of an orthopedic internal fixation implant medical device provided in Embodiment 5.
Fig. 4 is a diagram of a cross-section of an orthopedic internal fixation implant medical device provided in Embodiment 7.
Fig. 5 is a diagram of a cross-section of an orthopedic internal fixation implant medical device provided in Embodiment 9.
Fig. 6 is a diagram of a cross-section of an orthopedic internal fixation implant medical device provided in Embodiment 10.

### Detailed Description of the Invention

Exemplary implementations of the present invention will be described in more detail below with reference to the accompanying drawings. While exemplary implementations of the present invention have been illustrated in the accompanying drawings, it is to be understood that the present invention may be embodied in various forms and should not be construed as limited to the implementations set forth herein. Instead, these embodiments are provided to enable a more thorough understanding of the present invention and to fully convey the scope of the present invention to those skilled in the art.

It is to be understood that the terms used herein are for the purpose of describing particular example implementations only and are not intended to be limiting. As used herein, singular forms "a", "an", and "the" are intended to include plural forms as well, unless the context clearly indicates otherwise. Terms "include", "comprise", "contain", and "have" are inclusive and therefore specify the presence of stated features, steps, operations, elements, and/or components, but do not preclude the presence or addition of one or more other features, steps, operations, elements, components, and/or combination thereof. The method steps, processes, and operations described herein are not to be construed as necessarily requiring their performance in the particular order described or illustrated, unless an order of performance is expressly stated. It should also be understood that additional or alternative steps may be used.

The embodiment provides an orthopedic internal fixation implant medical device, including an iron matrix and a filling material including polylactic acid and an alkaline substance, where the polylactic acid has a weight-average molecular weight of Mw kDa, the alkaline substance includes a metal element, a mass ratio of the metal element in the alkaline substance to the polylactic acid is p, and the p and the Mw satisfy a formula of 2 M_{w}^-0.8 ≤ p ≤ 30 M_{w}^-0.5. The alkaline substance is a combination of hydroxyapatite and at least one of magnesium, magnesium alloy, zinc, zinc alloy, magnesium oxide, magnesium hydroxide, zinc oxide, zinc hydroxide, magnesium carbonate, zinc carbonate, magnesium phosphate, zinc phosphate, sodium carbonate, sodium bicarbonate, calcium oxide, calcium hydroxide, calcium carbonate, and calcium phosphate. The formula is written as that the mass ratio p of the metal element in the alkaline substance to the polylactic acid is greater than or equal to 2 times the -0.8 power of the weight-average molecular weight M_{w} of the polylactic acid, and the mass ratio p of the metal element in the alkaline substance to the polylactic acid is less than or equal to 30 times the -0.5 power of the weight-average molecular weight M_{w} of the polylactic acid.

The iron matrix can provide sufficient mechanical support to solve the problem of insufficient mechanical properties of polylactic acid orthopedic internal fixation medical devices and magnesium alloy orthopedic internal fixation medical devices. The alkaline substance can neutralize the acidity of the degradation product of the polylactic acid in the early stage. In response to p and M_{w} satisfying the formula, it can not only keep the local pH stable and make the pH neutral to reduce the inflammatory reactions, which is beneficial to bone repair, but also slow down the early degradation rate of the iron matrix to maintain good mechanical properties during bone healing.

In an embodiment, the polylactic acid has a weight-average molecular weight of 5 kDa to 1000 kDa. Preferably, the polylactic acid has a weight-average molecular weight of 100 kDa to 500 kDa, so that the early acidity of the polylactic acid is weaker, and at the same time, the degradation cycle becomes longer, which is beneficial to the accelerated degradation of the iron matrix in the later stage.

The alkaline substance is a combination of hydroxyapatite and at least one of magnesium oxide, magnesium hydroxide, zinc oxide, zinc hydroxide, magnesium carbonate, zinc carbonate, magnesium phosphate, zinc phosphate, sodium carbonate, sodium bicarbonate, calcium oxide, calcium hydroxide, calcium carbonate, and calcium phosphate, where the hydroxyapatite is used for enhancing bioactivity and promoting bone healing. Among them, the above oxides or hydroxides, or weak acid and strong base salts can avoid the hydrogen bubbles formed by the reaction of such metals as magnesium, magnesium alloy, zinc, and zinc alloy with the polylactic acid, which is conducive to tissue growth and repair. At the same time, the alkaline substance, with lower alkalinity than that of oxides or hydroxides, has better biocompatibility.

In an embodiment, the alkaline substance is one or more of a powder, granule, block, or rod, which facilitates the addition of the alkaline substance to the filling material in various states.

In an embodiment, the alkaline substance includes hydroxyapatite, a mass of which is 1% to 10% of that of the orthopedic internal fixation medical device.

In an embodiment, the polylactic acid is poly-dl-lactic acid or poly-L-lactic acid.

In an embodiment, the iron matrix is pure iron, low alloy steel, or iron-based alloy with a carbon content of not more than 2.5 wt.%. The low alloy steel is an alloy steel with a total amount of alloying elements of less than 5%. Preferably, the iron matrix is nitrided iron with a carbon content of less than or equal to 0.25%, belonging to an iron-based alloy with a carbon content of not more than 2.5 wt.%. Nitrided iron has better mechanical properties.

In the embodiment of the present invention, the connection relationship between the iron matrix and the filling material has various forms. The iron matrix is a hollow structure, and the filling material is filled inside the iron matrix; or the iron matrix is a mesh skeleton structure, and the filling material is filled in meshes of the mesh skeleton structure; or the iron matrix is a hollow space skeleton structure, and the filling material is filled inside the hollow space of the iron matrix; or a groove or hole is disposed on a surface of the iron matrix, and the filling material is filled in the groove or hole of the iron matrix; or the filling material is coated on the surface of the iron matrix.

The shape of the iron matrix is a nail, mesh, plate, rod, cylinder, cube, or cone.

The orthopedic internal fixation implant medical instrument in the embodiments of the present invention may be a bone nail, bone plate, bone rod, or bone mesh.

The above medical devices are further illustrated by specific embodiments below.

The test methods involved in the following embodiments are as follows:
1. Determination of weight-average molecular weight of the polylactic acid
   Detection is performed using a GPC-multi-angle laser light scattering instrument from Wyatt, USA coupled with a molecular weight test system. The test system includes liquid phase pump and sample injector, Agilent PL MIXED-C GPC column (dimension: 7.5 x 300 mm, 5 micron) from Agilent, USA, and multi-angle laser light scattering instrument and differential detector from Wyatt, USA. The detection conditions are as follows:
   Mobile phase: tetrahydrofuran; pump flow rate: 1 mL/min; injection volume: 100 µL; laser wavelength: 663.9 nm; and test temperature: 35°C.
2. Polylactic acid mass
   The weighed orthopedic internal fixation implant medical device is placed in a solvent that can dissolve the polylactic acid (such as ethyl acetate, chloroform, and the like), filtered after performing ultrasonic cleaning for 30 minutes, and weighed after drying the filtrate. The mass difference before and after cleaning is a mass of the polylactic acid.
3. Phase identification of alkaline substances
   The phase of alkaline substances is determined by using XRD to detect the orthopedic internal fixation implant medical device, and comparing the standard chromatograms of iron, hydroxyapatite, magnesium, zinc, magnesium oxide, zinc oxide, magnesium hydroxide, zinc hydroxide, magnesium carbonate, zinc carbonate, magnesium phosphate, zinc phosphate, sodium carbonate, sodium bicarbonate, calcium oxide, calcium hydroxide, calcium carbonate, and calcium phosphate.
4. Masses of metal elements in alkaline substances
   After the orthopedic internal fixation implant medical device is digested with nitric acid, AAS is used to determine the concentration of magnesium ions or zinc ions or calcium ions or sodium ions in the digestion solution. The masses of the metal elements in alkaline substances in the orthopedic internal fixation implant medical device can be obtained through calculation.
5. Bending strength
   The three-point bending strength of the samples are tested adopting C43.504 universal material testing machine manufactured by MTS and according to YBT5349-2006 metal material bending mechanical property test standard.
6. Degradation rate of iron

The corrosion of the iron-based matrix is evaluated through the mass loss rate after the absorbable iron-based orthopedic internal fixation implant medical device is implanted into an animal. The included specific steps are as follows: the absorbable iron-based orthopedic internal fixation implant medical device with an iron-based matrix mass of M0 is implanted into the animal. At predetermined observation time points, such as 3 months, 6 months, 12 months, and the like, the device and its surrounding tissues are removed before soaked in a 1 mol/L sodium hydroxide solution, to remove the remaining degradable polyester and digest the tissues. The device is then removed from the sodium hydroxide solution and placed in a 3% tartaric acid solution for ultrasound, to remove all the corrosion products and other alkaline materials attached to the device or dissolve in a good solvent. The remaining device is removed, dried, and weighed to a mass of M1. The mass loss rate of the iron-based matrix at the observation time point is (M0-M1)/M0 x 100%.

It is considered that the iron-based matrix is not corroded in a time zone from the implantation time point to the observation time point in response to the mass loss rate W of the iron-based matrix at a certain observation time point is less than 5%. It is considered that the iron-based matrix is completely corroded in response to the mass loss rate W of the iron-based matrix at a certain observation time point is greater than or equal to 90%, the time zone from the implantation time point to the observation time point being a corrosion cycle of the iron-based matrix.

### 7. Local pH value after degradation

The orthopedic internal fixation implanted medical device is immersed in a PBS solution (pH = 7.4 ± 0.1) for corrosion at 37°C for 7 days before being removed, and the pH value on the surface of the device is immediately detected with a pH test paper.

### Embodiment 1

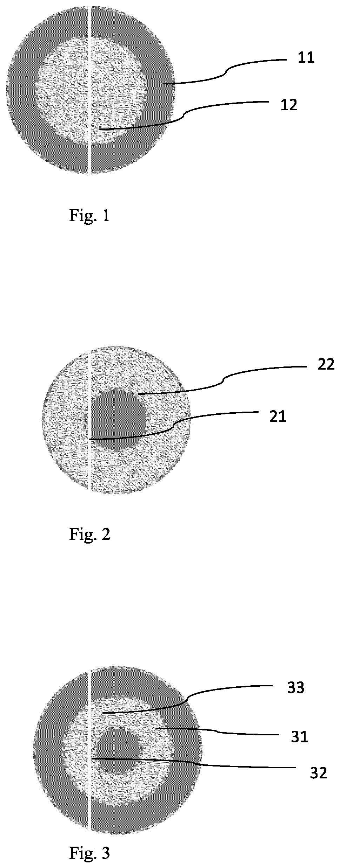

Pure iron was cast into a hollow nail with holes on the surface to obtain an iron matrix 11; pure magnesium powder and hydroxyapatite powder were dispersed in molten poly-L-lactic acid, and the mixture 12 was filled in the hollow iron matrix 11 to prepare an absorbable iron-based bone nail, the cross-section of which was shown in Fig. 1. Poly-L-lactic acid had a weight-average molecular weight of 1000 kDa, and a mass ratio of a magnesium element to poly-L-lactic acid was 0.94. The mass of hydroxyapatite was 1% of that of the whole orthopedic internal fixation implant medical device.

The initial bending strength of the bone nail was 350 MPa. The bone nail was implanted in the animal and removed after 6 months with 10% iron degradation.

The orthopedic internal fixation implanted medical device was immersed in a PBS solution for a water bath at 37°C for 7 days before being removed, and the pH value on the surface of the device was immediately detected as 7 to 8 with a pH test paper.

### Embodiment 2

Pure iron was cast into a hollow nail with holes on the surface to obtain an iron matrix; magnesium oxide particles and hydroxyapatite powder were dispersed in molten poly-L-lactic acid, and the mixture was filled in the hollow iron matrix to prepare an absorbable iron-based bone nail. Poly-L-lactic acid had a weight-average molecular weight of 1000 kDa, and a mass ratio of a magnesium element to poly-L-lactic acid was 0.008. The mass of hydroxyapatite was 1% of that of the whole orthopedic internal fixation implant medical device.

The initial bending strength of the bone nail was 350 MPa. The bone nail was implanted in the animal and removed after 6 months with 17% iron degradation.

The orthopedic internal fixation implanted medical device was immersed in a PBS solution for a water bath at 37°C for 7 days before being removed, and the pH value on the surface of the device was immediately detected as 6 to 7 with a pH test paper.

### Embodiment 3

Pure iron was cast into a nail, and the strength thereof was enhanced by ion nitriding to obtain a nail iron matrix 21; a bulk zinc alloy and hydroxyapatite powder were dispersed in molten poly-di-lactic acid, and the mixture 22 was coated on the surface of the nail iron matrix 21 to prepare an absorbable iron-based bone nail, the cross-section of which was shown in Fig. 2. poly-dl-lactic acid had a weight-average molecular weight of 500 kDa, and a mass ratio of a zinc element to poly-dl-lactic acid was 1.3. The mass of hydroxyapatite was 3% of that of the whole orthopedic internal fixation implant medical device.

The initial bending strength of the bone nail was 450 MPa. The bone nail was implanted in the animal and removed after 6 months with 15% iron degradation.

The orthopedic internal fixation implanted medical device was immersed in a PBS solution for a water bath at 37°Cfor 7 days before being removed, and the pH value on the surface of the device was immediately detected as 7 to 8 with a pH test paper.

### Embodiment 4

Pure iron was cast into a nail with grooves on the surface, and the strength thereof was enhanced by ion nitriding to obtain a nail iron matrix; magnesium hydroxide powder and hydroxyapatite powder were dispersed in molten poly-L-lactic acid, and the mixture was coated on the surface of the nail iron matrix to prepare an absorbable iron-based bone nail. Poly-L-lactic acid had a weight-average molecular weight of 500 kDa, and a mass ratio of a magnesium element to poly-L-lactic acid was 0.014. The mass of hydroxyapatite was 3% of that of the whole orthopedic internal fixation implant medical device.

The initial bending strength of the bone nail was 420 MPa. The bone nail was implanted in the animal and removed after 6 months with 18% iron degradation.

The orthopedic internal fixation implanted medical device was immersed in a PBS solution for a water bath at 37°C for 7 days before being removed, and the pH value on the surface of the device was immediately detected as 6 to 7 with a pH test paper.

### Embodiment 5

Low alloy steel was cast into a hollow nail 31, the interior of which also included an iron support rod 32 parallel to the iron nail, and the cross-section was shown in Fig. 3. The iron nail was cut into a hollow tubular structure with a laser cutter. Magnesium oxide powder and hydroxyapatite powder were dispersed in molten poly-dl-lactic acid, and the mixture 33 was filled inside an iron matrix to obtain an absorbable iron-based bone nail. Poly-dl-lactic acid had a weight-average molecular weight of 100 kDa, and a mass ratio of a magnesium element to poly-dl-lactic acid was 0.05. The mass of hydroxyapatite was 10% of that of the whole orthopedic internal fixation implant medical device.

The initial bending strength of the bone nail was 380 MPa. The bone nail was implanted in the animal and removed after 6 months with 25% iron degradation.

The orthopedic internal fixation implanted medical device was immersed in a PBS solution for a water bath at 37°C for 7 days before being removed, and the pH value on the surface of the device was immediately detected as 6 to 7 with a pH test paper.

### Embodiment 6

Low alloy steel was cast into a hollow rod with holes on the surface to obtain a rod iron matrix. A small zinc rod and hydroxyapatite powder were dispersed in molten poly-dl-lactic acid, and the mixture was filled inside an iron rod to obtain an absorbable iron-based bone rod. poly-dl-lactic acid had a weight-average molecular weight of 100 kDa, and a mass ratio of a zinc element to poly-dl-lactic acid was 3. The mass of hydroxyapatite was 10% of that of the whole orthopedic internal fixation implant medical device.

The initial bending strength of the bone rod was 480 MPa. The bone nail was implanted in the animal and removed after 6 months with 18% iron degradation.

The orthopedic internal fixation implanted medical device was immersed in a PBS solution for a water bath at 37°C for 7 days before being removed, and the pH value on the surface of the device was immediately detected as 7 to 8 with a pH test paper.

### Embodiment 7

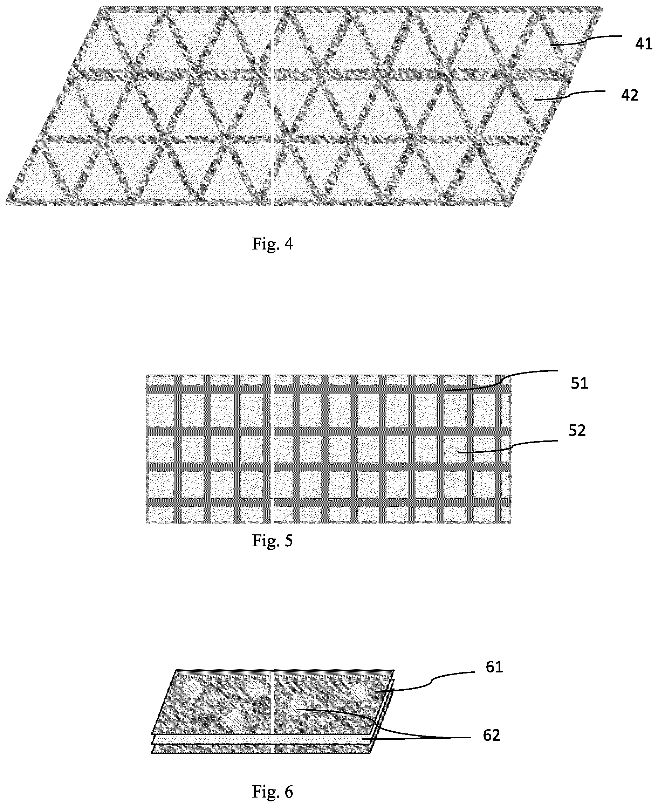

A piece of pure iron sheet was taken and cut into a mesh with a laser cutter to obtain a mesh iron matrix 41. Zinc oxide powder and hydroxyapatite powder were dispersed in molten poly-dl-lactic acid, and the mixture 42 was coated on the surface of the iron mesh and in the meshes to obtain an iron-based resorbable bone mesh, as shown in Fig. 4. poly-dl-lactic acid had a weight-average molecular weight of 5 kDa, and a mass ratio of a zinc element to poly-dl-lactic acid was 0.55. The mass of hydroxyapatite was 6% of that of the whole orthopedic internal fixation implant medical device.

The initial bending strength of the bone mesh was 350 MPa. The bone nail was implanted in the animal and removed after 6 months with 20% iron degradation.

The orthopedic internal fixation implanted medical device was immersed in a PBS solution for a water bath at 37°C for 7 days before being removed, and the pH value on the surface of the device was immediately detected as 6 to 7 with a pH test paper.

### Embodiment 8

A piece of pure iron sheet was taken and cut into a mesh with a laser cutter to obtain a mesh iron matrix. Zinc powder and hydroxyapatite powder were dispersed in molten poly-dl-lactic acid, and the mixture was coated on the surface of the iron mesh and in the meshes to obtain an iron-based resorbable bone mesh. poly-dl-lactic acid had a weight-average molecular weight of 5 kDa, and a mass ratio of a zinc element to poly-dl-lactic acid was 13.4. The mass of hydroxyapatite was 6% of that of the whole orthopedic internal fixation implant medical device.

The initial bending strength of the bone mesh was 500 MPa. The bone nail was implanted in the animal and removed after 6 months with 14% iron degradation.

The orthopedic internal fixation implanted medical device was immersed in a PBS solution for a water bath at 37°C for 7 days before being removed, and the pH value on the surface of the device was immediately detected as 7 to 8 with a pH test paper.

### Embodiment 9

An iron wire with a zinc layer on a hollow surface was taken and woven into an iron mesh to obtain a mesh iron matrix 51. Zinc oxide powder and hydroxyapatite powder were dispersed in molten poly-dl-lactic acid, and the mixture 52 was coated on the surface of the iron mesh and in the meshes to obtain an absorbable iron-based bone mesh, as shown in Fig. 5. Poly-dl-lactic acid had a weight-average molecular weight of 200 kDa, and a mass ratio of a zinc element to poly-dl-lactic acid was 0.03. The mass of hydroxyapatite was 7% of that of the whole orthopedic internal fixation implant medical device.

The initial bending strength of the bone mesh was 420 MPa. The bone nail was implanted in the animal and removed after 6 months with 27% iron degradation.

The orthopedic internal fixation implanted medical device was immersed in a PBS solution for a water bath at 37°C for 7 days before being removed, and the pH value on the surface of the device was immediately detected as 6 to 7 with a pH test paper.

### Embodiment 10

Pure iron was cast into a hollow iron plate 61 with holes on the surface; magnesium oxide powder and hydroxyapatite powder were dispersed in molten poly-dl-lactic acid, and the mixture 62 was filled in the middle of the iron plate 61 to obtain an absorbable iron-based bone plate, as shown in Fig. 6. Poly-dl-lactic acid had a weight-average molecular weight of 200 kDa, and a mass ratio of a magnesium element to poly-di-lactic acid was 2.1. The mass of hydroxyapatite was 7% of that of the whole orthopedic internal fixation implant medical device.

The initial bending strength of the bone mesh was 600 MPa. The bone nail was implanted in the animal and removed after 6 months with 20% iron degradation.

The orthopedic internal fixation implanted medical device was immersed in a PBS solution for a water bath at 37°C for 7 days before being removed, and the pH value on the surface of the device was immediately detected as 7 to 8 with a pH test paper.

### Comparative embodiment 1

A poly-L-lactic acid absorbable bone nail, with the molecular weight of poly-L-lactic acid being 500 kDa.

The initial bending strength of the bone nail was 150 MPa.

The absorbable bone nail was immersed in a PBS solution for a water bath at 37°C for 7 days before being removed, and the pH value on the surface of the device was immediately detected as 4 to 5 with a pH test paper.

## Claims

1. An orthopedic internal fixation implant medical device, **characterized by** comprising an iron matrix and a filling material, the filling material comprising polylactic acid and an alkaline substance, wherein the polylactic acid has a weight-average molecular weight of M_{w} kDa, the alkaline substance comprises a metal element, a mass ratio of the metal element in the alkaline substance to the polylactic acid is p, and the p and the M_{w} satisfy a formula of 2 M_{w}^-0.8 ≤ p ≤ 30 M_{w}^-0.5;
wherein the alkaline substance is a combination of hydroxyapatite and at least one of magnesium, magnesium alloy, zinc, zinc alloy, magnesium oxide, magnesium hydroxide, zinc oxide, zinc hydroxide, magnesium carbonate, zinc carbonate, magnesium phosphate, zinc phosphate, sodium carbonate, sodium bicarbonate, calcium oxide, calcium hydroxide, calcium carbonate, and calcium phosphate.

2. The orthopedic internal fixation implant medical device according to claim 1, **characterized in that** the polylactic acid has a weight-average molecular weight of 5 kDa to 1000 kDa.

3. The orthopedic internal fixation implant medical device according to claim 1, **characterized in that** the alkaline substance is one or more of a powder, granule, block or rod.

4. The orthopedic internal fixation implant medical device according to claim 3, **characterized in that** a mass of the hydroxyapatite is 1% to 10% of that of the orthopedic internal fixation medical device.

5. The orthopedic internal fixation implant medical device according to claim 1, **characterized in that** the polylactic acid is poly-dl-lactic acid or poly-L-lactic acid.

6. The orthopedic internal fixation implant medical device according to claim 1, **characterized in that** the iron matrix is a hollow structure, and the filling material is filled inside the iron matrix; or
the iron matrix is a mesh skeleton structure, and the filling material is filled in meshes of the mesh skeleton structure; or
the iron matrix is a hollow space skeleton structure, and the filling material is filled inside the hollow space of the iron matrix; or
a groove or hole is disposed on a surface of the iron matrix, and the filling material is filled in the groove or hole of the iron matrix; or
the filling material is coated on the surface of the iron matrix.

7. The orthopedic internal fixation implant medical device according to claim 1, **characterized in that** the orthopedic internal fixation implant medical device is a bone nail, bone plate, bone rod, or bone mesh.

8. The orthopedic internal fixation implant medical device according to claim 1, **characterized in that** the iron matrix is pure iron, low alloy steel, or iron-based alloy with a carbon content of not more than 2.5 wt.%.

## Patentansprüche

1. Medizinische Implantatvorrichtung zur orthopädischen internen Fixation, **dadurch gekennzeichnet, dass** sie eine Eisenmatrix und ein Füllmaterial umfasst, wobei das Füllmaterial Polymilchsäure und einen alkalischen Stoff umfasst, wobei die Polymilchsäure ein gewichtsgemitteltes Molekulargewicht von M_{w} kDa aufweist, der alkalische Stoff ein Metallelement umfasst, ein Massenverhältnis des Metallelements im alkalischen Stoff zu der Polymilchsäure p beträgt und p und M_{w} einer Formel von 2 M_{w}^-0,8 ≤ p ≤ 30 M_{w}^-0,5 genügen,
wobei es sich bei dem alkalischen Stoff um eine Kombination aus Hydroxylapatit und wenigstens einem von Magnesium, einer Magnesiumlegierung, Zink, einer Zinklegierung, Magnesiumoxid, Magnesiumhydroxid, Zinkoxid, Zinkhydroxid, Magnesiumcarbonat, Zinkcarbonat, Magnesiumphosphat, Zinkphosphat, Natriumcarbonat, Natriumbicarbonat, Calciumoxid, Calciumhydroxid, Calciumcarbonat und Calciumphosphat handelt.

2. Medizinische Implantatvorrichtung zur orthopädischen internen Fixation nach Anspruch 1, **dadurch gekennzeichnet, dass** die Polymilchsäure ein gewichtsgemitteltes Molekulargewicht von 5 kDa bis 1000 kDa aufweist.

3. Medizinische Implantatvorrichtung zur orthopädischen internen Fixation nach Anspruch 1, **dadurch gekennzeichnet, dass** der alkalische Stoff ein Pulver und/oder ein Granulat und/oder ein Block und/oder ein Stab ist.

4. Medizinische Implantatvorrichtung zur orthopädischen internen Fixation nach Anspruch 3, **dadurch gekennzeichnet, dass** eine Masse des Hydroxylapatits 1% bis 10% derjenigen der medizinischen Vorrichtung zur orthopädischen internen Fixation beträgt.

5. Medizinische Implantatvorrichtung zur orthopädischen internen Fixation nach Anspruch 1, **dadurch gekennzeichnet, dass** die Polymilchsäure Poly-D,L-Milchsäure oder Poly-L-Milchsäure ist.

6. Medizinische Implantatvorrichtung zur orthopädischen internen Fixation nach Anspruch 1, **dadurch gekennzeichnet, dass** die Eisenmatrix eine Hohlstruktur ist und das Füllmaterial in die Eisenmatrix gefüllt ist oder
die Eisenmatrix eine Netzskelettstruktur ist und das Füllmaterial in Maschen der Netzskelettstruktur gefüllt ist oder
die Eisenmatrix eine Hohlraumskelettstruktur ist und das Füllmaterial in den Hohlraum der Eisenmatrix gefüllt ist oder
eine Rille oder ein Loch an einer Oberfläche der Eisenmatrix angeordnet ist und das Füllmaterial in die Rille oder das Loch der Eisenmatrix gefüllt ist oder
die Oberfläche der Eisenmatrix mit dem Füllmaterial beschichtet ist.

7. Medizinische Implantatvorrichtung zur orthopädischen internen Fixation nach Anspruch 1, **dadurch gekennzeichnet, dass** die medizinische Implantatvorrichtung zur orthopädischen internen Fixation ein Knochennagel, eine Knochenplatte, ein Knochenstab oder ein Knochennetz ist.

8. Medizinische Implantatvorrichtung zur orthopädischen internen Fixation nach Anspruch 1, **dadurch gekennzeichnet, dass** die Eisenmatrix reines Eisen, niedriglegierter Stahl oder eine Legierung auf Eisenbasis mit einem Kohlenstoffgehalt von nicht mehr als 2,5 Gew.-% ist.

## Revendications

1. Dispositif d'implant médical pour une fixation interne orthopédique, **caractérisé en ce qu'**il comprend une matrice en fer et un matériau de remplissage, le matériau de remplissage comprenant de l'acide polylactique et une substance alcaline, l'acide polylactique présentant un poids moléculaire moyen en poids de M_{w} kDa, la substance alcaline comprenant un élément métallique, un rapport massique entre l'élément métallique dans la substance alcaline et l'acide polylactique étant p, et p et M_{w} satisfaisant à une formule de 2 M_{w}^-0,8 ≤ p ≤ 30 M_{w}^-0,5,
la substance alcaline étant une combinaison d'hydroxyapatite et d'au moins un élément parmi le magnésium, un alliage de magnésium, le zinc, un alliage de zinc, l'oxyde de magnésium, l'hydroxyde de magnésium, l'oxyde de zinc, l'hydroxyde de zinc, le carbonate de magnésium, le carbonate de zinc, le phosphate de magnésium, le phosphate de zinc, le carbonate de sodium, le bicarbonate de sodium, l'oxyde de calcium, l'hydroxyde de calcium, le carbonate de calcium, le phosphate de calcium.

2. Dispositif d'implant médical pour une fixation interne orthopédique selon la revendication 1, **caractérisé en ce que** l'acide polylactique présente un poids moléculaire moyen en poids compris entre 5 kDa et 1 000 kDa.

3. Dispositif d'implant médical pour une fixation interne orthopédique selon la revendication 1, **caractérisé en ce que** la substance alcaline est une poudre et/ou des granulés et/ou un bloc et/ou une tige.

4. Dispositif d'implant médical pour une fixation interne orthopédique selon la revendication 3, **caractérisé en ce que** la masse de l'hydroxyapatite représente 1 % à 10 % de celle du dispositif médical pour une fixation interne orthopédique.

5. Dispositif d'implant médical pour une fixation interne orthopédique selon la revendication 1, **caractérisé en ce que** l'acide polylactique est de l'acide poly-dl-lactique ou de l'acide poly-L-lactique.

6. Dispositif d'implant médical pour une fixation interne orthopédique selon la revendication 1, **caractérisé en ce que** la matrice en fer est une structure creuse, et le matériau de remplissage est rempli dans la matrice en fer, ou
la matrice en fer est une structure squelettique maillée, et le matériau de remplissage est rempli dans les mailles de la structure squelettique maillée, ou
la matrice en fer est une structure squelettique à espace creux, et le matériau de remplissage est rempli dans l'espace creux de la matrice en fer, ou
une rainure ou un trou est aménagé(e) sur une surface de la matrice en fer, et le matériau de remplissage est rempli dans la rainure ou le trou de la matrice en fer, ou
la surface de la matrice en fer est revêtue du matériau de remplissage.

7. Dispositif d'implant médical pour une fixation interne orthopédique selon la revendication 1, **caractérisé en ce que** le dispositif d'implant médical pour une fixation interne orthopédique est un clou osseux, une plaque osseuse, une tige osseuse ou un treillis osseux.

8. Dispositif d'implant médical pour une fixation interne orthopédique selon la revendication 1, **caractérisé en ce que** la matrice en fer est du fer pur, de l'acier faiblement allié ou un alliage à base de fer à teneur en carbone ne dépassant pas 2,5 % en poids.
